(19) Europäisches Patentamt
European Patent Office
Office européen des brevets

(11) Veröffentlichungsnummer: **0 062 837**
**A1**

# (12) EUROPÄISCHE PATENTANMELDUNG

(21) Anmeldenummer: 82102696.0

(22) Anmeldetag: 31.03.82

(51) Int. Cl.³: C 07 C 121/30
H 01 B 1/12, H 01 L 31/02
C 07 C 103/153, C 07 C 15/50

(30) Priorität: 09.04.81 DE 3114342

(43) Veröffentlichungstag der Anmeldung:
20.10.82 Patentblatt 82/42

(84) Benannte Vertragsstaaten:
BE DE FR GB NL

(71) Anmelder: BASF Aktiengesellschaft
Carl-Bosch-Strasse 38
D-6700 Ludwigshafen(DE)

(72) Erfinder: Naarmann, Herbert, Dr.
Haardtblick 15
D-6719 Wattenheim(DE)

(72) Erfinder: Feichtmayr, Franz, Dr.
Mundenheimer Strasse 158
D-6700 Ludwigshafen(DE)

(72) Erfinder: Paust, Joachim, Dr.
Ringstrasse 3
D-6708 Neuhofen(DE)

(72) Erfinder: Penzien, Klaus, Dr.
Bensheimer Ring 18
D-6710 Frankenthal(DE)

## (54) Verfahren zur Herstellung elektrisch leitfähiger Polyen-Verbindungen mit elektronenanziehenden Seitengruppen und deren Verwendung in der Elektrotechnik und zur antistatischen Ausrüstung von Kunststoffen.

(57) Die Erfindung betrifft ein Verfahren zur Herstellung von elektrisch Leitfähigen polymeren Systemen mit elektrischen Leitfähigkeitswerten größer als $10^{-4}$ S/cm aus Polyen-Verbindungen, die mindestens einmal das Kettenglied mit der Formel:

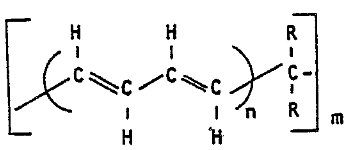

enthalten, wobei die Kettenzahlen n und m jeweils größer als 1, vorzugsweise 2 bis 20, sind und R eine Nitril-, Säureamid- oder Phenylgruppe ist, und die unter Ausschluß von Wasserfeuchtigkeit und von Sauerstoff mit 0,03 bis 0,9 Molprozent einer starken Lewis-Säure mit $pk_s$-Werten von −10 bis +4 oder eines Alkalimetalls behandelt werden.

Die erhaltenen elektrisch leitfähigen polymeren Systeme können in der Elektrotechnik zur Herstellung von Sonnenzellen, zur Umwandlung und Fixierung von Strahlung, zur Herstellung elektrischer und magnetischer Schalter und elektrischer Speicher und zur antistatischen Ausrüstung von Kunststoffen verwendet werden.

EP 0 062 837 A1

# Verfahren zur Herstellung elektrisch leitfähiger Polyen-Verbindungen mit elektronenanziehenden Seitengruppen und deren Verwendung in der Elektrotechnik und zur antistatischen Ausrüstung von Kunststoffen

Die Erfindung betrifft ein Verfahren zur Herstellung von elektrisch leitfähigen polymeren Systemen mit elektrischen Leitfähigkeitswerten größer als $10^{-4}$ S/cm aus Polyen-Verbindungen, die unter Ausschluß von Wasserfeuchtigkeit und von Sauerstoff mit 0,03 bis 0,9 Molprozent, bezogen auf die eingesetzte Polyen-Verbindung, einer starken Lewis-Säure mit $pk_s$-Werten von -10 bis +4 oder eines Alkalimetalls behandelt werden.

Bei derartigen Verfahren sind Zusätze erforderlich, um eine Erhöhung der elektrischen Leitfähigkeitswerte bei Polyen-Verbindungen zu bewirken.

Aus der DE-OS 29 47 797 ist es bereits bekannt, die elektrische Leitfähigkeit von Polyen-Verbindungen, die mindestens einmal das Kettenglied der Formel:

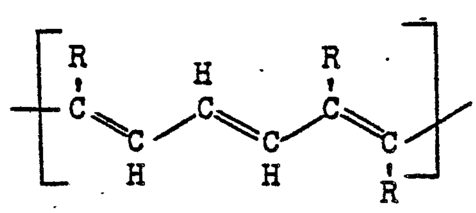

in der R ein Wasserstoffatom oder eine Methylgruppe bedeutet und insgesamt mindestens sieben aliphatische Doppelbindungen enthalten, zu erhöhen, indem man unter Ausschluß von Wasserfeuchtigkeit oder von Sauerstoff mit 0,03 bis 0,9 Molprozent einer starken Lewis-Säure wie $AsF_5$, $SbF_5$, $FSO_3H$, $HClO_4$, $NO^+AsF_6^-$ oder eines Alkalimetalls behandelt. Die erhaltenen leitfähigen Polyen-Verbindungen sind außerordentlich empfindlich gegenüber der Einwirkung von Feuchtigkeit und Luftsauerstoff.

Rss/P

Der Erfindung lag die Aufgabe zugrunde, elektrisch leitfähige Polyen-Verbindungen zu schaffen, die entsprechend dem Stand der Technik dotierbar sind und im Vergleich zu bekannten Verbindungen eine geringe Hydrolysetendenz aufweisen und zusätzlich eine erhöhte Stabilität gegenüber Luftsauerstoff aufweisen.

Diese Aufgabe wurde erfindungsgemäß dadurch gelöst, daß man Polyen-Verbindungen einsetzt, die mindestens einmal das Kettenglied mit der Formel:

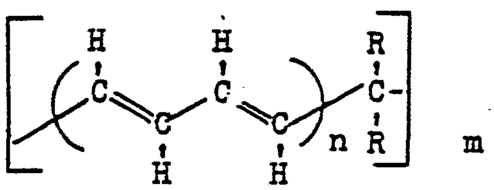

enthalten, wobei die Kettenzahlen n und m jeweils größer als 1, bevorzugt 2 bis 20 sind und R eine Nitril-, Säureamid- oder Phenylgruppe ist.

Unter elektrisch leitfähigen polymeren Systemen mit elektrischen Leitfähigkeitswerten größer als $10^{-4}$ S/cm werden Substanzen verstanden, die nach der Meßmethode von F.Beck, Berichte der Bunsengesellschaft 68 (1964), Seiten 558 bis 567 eine elektrische Leitfähigkeit von mehr als $10^{-4}$ S/cm aufweisen.

Die Polyen-Verbindungen werden entsprechend dem in der DE-OS 29 47 797 beschriebenen Verfahren unter Ausschluß von Wasserfeuchtigkeit und von Sauerstoff mit 0,03 bis 0,9 Molprozent, bezogen auf die eingesetzte Polyen-Verbindung, einer starken Lewis-Säure mit $pk_s$-Werten von -10 bis +4 oder eines Alkalimetalls behandelt. Als Lewis-Säuren mit $pk_s$-Werten von -10 bis +4 eignen sich z.B. die Verbindungen $AsF_5$, $SbF_5$, $HClO_4$, $FSO_3H$, $ClO_2$, $NO^+SbF_6^-$, $NO_2^+SbF_6^-$,

$NO^+AsF_6^-$, $NO^+PF_6^-$, $NO_2^+PF_6^-$, $NO^+BF_4^-$, $NO_2^+BF_4^-$, $NO^+ClO_4^-$, $(CF_3)_2SO_4$, 2,4,6-Trinitrophenol, 2,4,6-Trinitrophenylsulfosäure oder 2,4,6-Trinitrobenzoesäure (n-Komplexierungsmittel).

Als Alkalimetalle verwendet man z.B. Natrium oder Kalium, die auch in Form von Lösungen, z.B. in Naphthalin oder α-Methylstyrol, eingesetzt werden können (p-Komplexierungsmittel).

Die Behandlung der Polyen-Verbindungen mit den Lewis-Säuren oder den Alkalimetallen führt man bei Temperaturen von -70 bis 150°C, vorzugsweise -10 bis 100°C, insbesondere 0 bis 30°C durch. Dabei wendet man die Säuren oder Alkalimetalle, die mit den Polyenen unter Komplexbildung die leitfähigen Verbindungen ergeben, in Mengen von 0,03 bis 0,9, vorzugsweise von 0,1 bis 0,5 Molprozent, bezogen auf die Polyen-Verbindungen an.

Das Einarbeiten der Zusätze erfolgt unter Ausschluß von Feuchtigkeit (Wasser) sowie Sauerstoff (Luft), es wird daher vorzugsweise unter Edelgas-Argonatmosphäre gearbeitet. Gegebenenfalls werden als Hilfsflüssigkeiten nicht-wäßrige Lösungsmittel, die unter den Verfahrensbedingungen nicht mit den Lewis-Säuren reagieren, wie Methylenchlorid, Tetrahydrofuran, Dimethoxyglykol, Nitromethan, Naphthalin oder α-Methylstyrol, eingesetzt. Die meist tieffarbigen elektrisch leitfähigen Polyene werden bei dem erfindungsgemäßen Verfahren in wenigen Sekunden bis Minuten gebildet. Gegebenenfalls verwendete Lösungsmittel werden zweckmäßig bei Temperaturen unter 100°C im Vakuum abgezogen.

Nach erfindungsgemäßem Verfahren werden Polyen-Verbindungen eingesetzt, die mindestens einmal das Kettenglied mit der Formel:

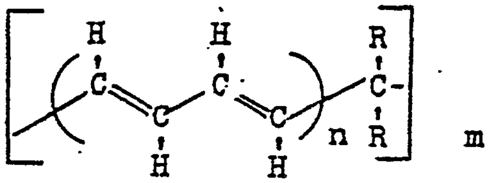

enthalten, wobei die Kettenzahlen n und m jeweils größer als 1, bevorzugt 2 bis 20, sind und R eine Nitril-, Säureamid- oder Phenylgruppe ist. Die erfindungsgemäß zu verwendenden Polyen-Derivate sind an sich bekannt und entsprechend der Literaturstelle DE-OS 16 18 733 herstellbar. Zweckmäßigerweise werden Polyendialdehyd, z.B. $C_4$-, $C_{10}$-, $C_{18}$-, $C_{30}$- und/oder $C_{40}$-Dialdehyd z.B. mit Bernsteinsäuredinitril und/oder 1,2-Diphenyläthan im Molverhältnis 1 : 1 in Gegenwart von Basen wie Natriumamid oder Kalium-tert.-butylat in Toluol bis zu acht Stunden auf Siedetemperatur erhitzt.

Für das erfindungsgemäße Verfahren sind die Polyen-Derivate I bis X besonders geeignet.

I

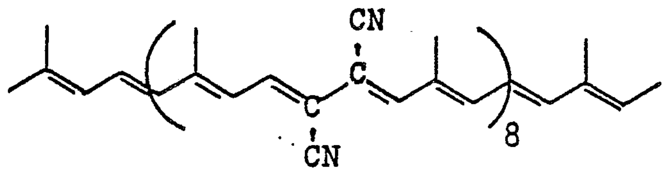

II

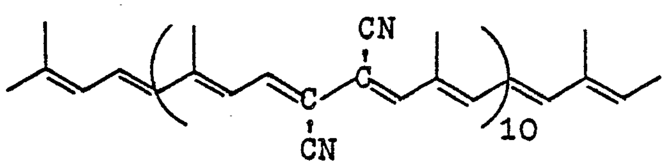

III

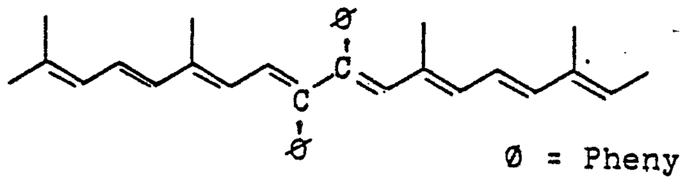

Ø = Phenylrest

IV

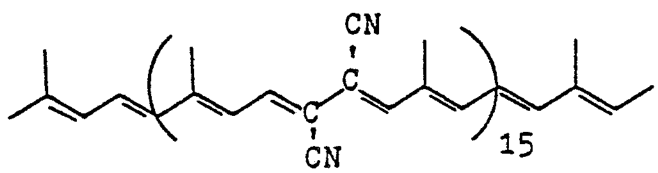

V

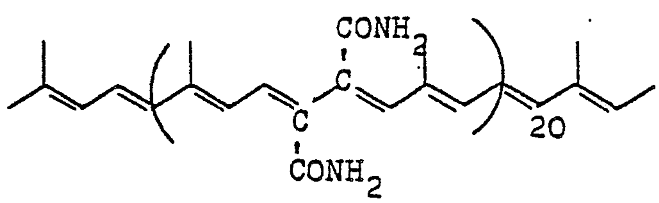

VI

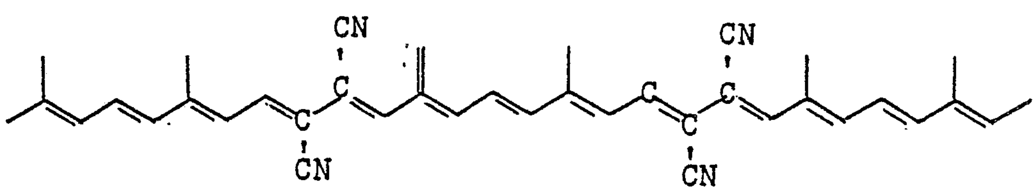

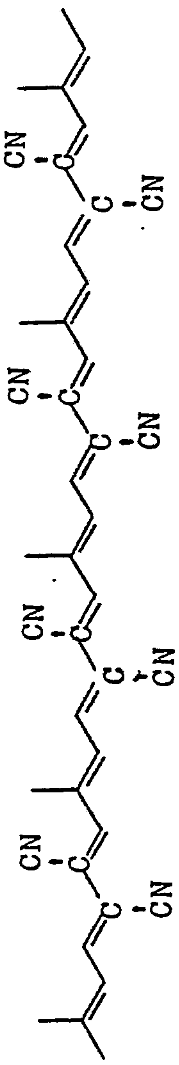

VII

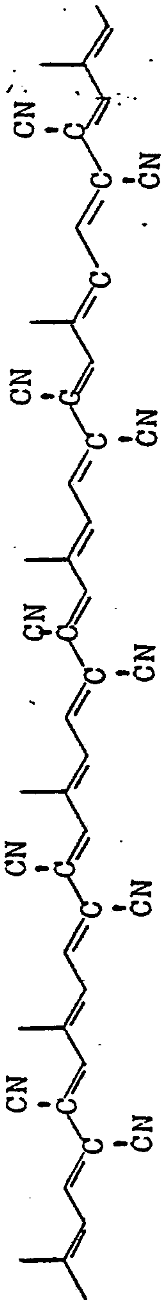

VIII

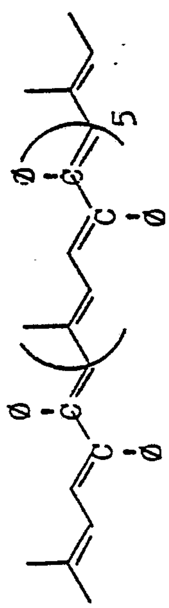

IX

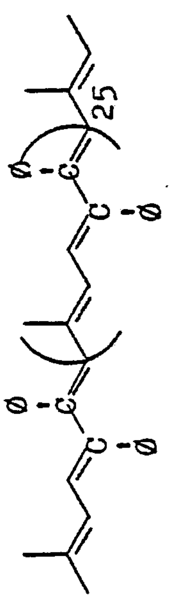

X

Die erfindungsgemäß hergestellten elektrisch leitfähigen Polyene sind zur antistatischen Ausrüstung von Kunststoffen zur Herstellung von Sonnenzellen, zur Umwandlung und Fixierung von Strahlung sowie zur Herstellung elektrischer und magnetischer Schalter und elektrischer Speicher verwendbar.

Die in den folgenden Beispielen genannten Teile sind Gewichtsteile.

Beispiele 1 bis 13

Man gibt zu 10 Teilen der erfindungsgemäßen Verbindung unter Argon bei Raumtemperatur und unter Ausschluß von Wasserfeuchtigkeit und Luftsauerstoff ein n- oder p-Komplexierungsmittel. Die Komplexbildung tritt sofort ein. Man erhält eine tiefblauschwarze kristalline Verbindung, die beim Messen in einer Leitfähigkeitsmeßzelle eine Leitfähigkeit von $> 10^{-4}$ S/cm aufweist. Die Leitfähigkeit des Ausgangspolyens betrug $< 10^{-10}$ S/cm.

Verfährt man wie in diesem Beispiel beschrieben, wobei man die in der Tabelle genannten Polyene und Zusätze verwendet, so werden Polyene mit den angegebenen Leitfähigkeiten erhalten.

| Beispiel | Polyen Art und Menge | Ausgangsleitfähigkeit bei 25°C [S/cm] | Dotierungsmittel Menge [Teile] und Art | Leitfähigkeit nach dem Dotieren bei 25°C [S/cm] |
|---|---|---|---|---|
| 2 | I 10 Teile | 1,0 . $10^{-10}$ | 15 $SbF_5$ ca. 0,1 Mol% | 1,3 . $10^{-5}$ |
| 3 | II 10 Teile | 1,4 . $10^{-10}$ | 15 $SbF_5$ ca. 0,1 Mol% | 1,5 . $10^{-4}$ |
| 4 | III 10 Teile | 1,4 . $10^{-10}$ | 18 $NO^+SbF_6^-$ ca. 0,1 Mol% | 1,9 . $10^{-4}$ |
| 5 | IV 10 Teile | 1,4 . $10^{-10}$ | 20 $NO_2^+SbF_6^-$ ca. 0,1 Mol% | 2,0 . $10^{-4}$ |
| 6 | V 10 Teile | 1,4 . $10^{-10}$ | 6 K ca. 0,2 Mol% | 0,1 . $10^{-3}$ |
| 7 | VI 10 Teile | 3,5 . $10^{-10}$ | 20 $SbF_5$ ca. 0,2 Mol% | 2,5 . $10^{-2}$ |
| 8 | VII 10 Teile | 2,4 . $10^{-10}$ | 15 $AsF_5$ ca. 0,1 Mol% | 3,5 . $10^{-1}$ |
| 9 | VIII 10 Teile | 1,0 . $10^{-10}$ | 21 $SbF_5$ ca. 0,2 Mol% | 4,9 . $10^{-4}$ |
| 10 | IX 10 Teile | 1,0 . $10^{-10}$ | 15 $AsF_5$ ca. 0,1 Mol% | 2,5 . $10^{-1}$ |
| 11 | I 10 Teile | 1,0 . $10^{-10}$ | 5 Na ca. 0,2 Mol% | 3,4 . $10^{-4}$ |
| 12 | I 10 Teile | 1,0 . $10^{-10}$ | 5 $AsF_5$ ca. 0,05 Mol% | 2,5 . $10^{-3}$ |
| 13 | I 10 Teile | 1,0 . $10^{-10}$ | 5 $NO_2^+PF_6^-$ ca. 0,05 Mol% | 1,8 . $10^{-4}$ |

Patentansprüche

1. Verfahren zur Herstellung von elektrisch leitfähigen polymeren Systemen mit elektrischen Leitfähigkeitswerten größer als $10^{-4}$ S/cm aus Polyen-Verbindungen, die unter Ausschluß von Wasserfeuchtigkeit und von Sauerstoff mit 0,03 bis 0,9 Molprozent, bezogen auf die eingesetzte Polyen-Verbindung, einer starken Lewis-Säure mit $pk_s$-Werten von -10 bis +4 oder eines Alkalimetalls behandelt werden, dadurch gekennzeichnet, daß man Polyen-Verbindungen einsetzt, die mindestens einmal das Kettenglied mit der Formel:

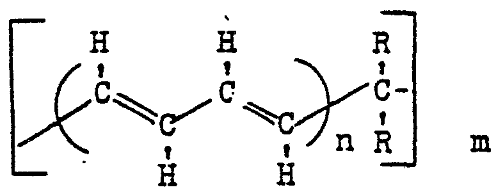

enthalten, wobei die Kettenzahlen n und m jeweils größer als 1 sind und R eine Nitril-, Säureamid- oder Phenylgruppe ist.

2. Verfahren nach Anspruch 1, dadurch gekennzeichnet, daß n und m jeweils 2 bis 20 sind.

3. Verwendung der nach Anspruch 1 hergestellten elektrisch leitfähigen polymeren Systeme in der Elektrotechnik zur Herstellung von Sonnenzellen, zur Umwandlung und Fixierung von Strahlung und zur Herstellung elektrischer und magnetischer Schalter und elektrischer Speicher.

4. Verwendung der nach Anspruch 1 hergestellten elektrisch leitfähigen polymeren Systeme zur antistatischen Ausrüstung von Kunststoffen.

0062837

Europäisches Patentamt

# EUROPÄISCHER RECHERCHENBERICHT

Nummer der Anmeldung

EP 82 10 2696

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 023 596 (BASF) * Ansprüche * | 1-4 | C 07 C 121/30<br>H 01 B 1/12<br>H 01 L 31/02<br>C 07 C 103/153<br>C 07 C 15/50 |
| | --- | | |
| P,A | EP-A-0 029 945 (BASF) * Ansprüche * & DE - A - 2 947 797 (Cat. D)(Veröffentlichungstag: 30-07-1981) | 1-4 | |
| | ----- | | |

| RECHERCHIERTE SACHGEBIETE (Int. Cl. 3) |
|---|
| H 01 B |

Der vorliegende Recherchenbericht wurde für alle Patentansprüche erstellt.

| Recherchenort | Abschlußdatum der Recherche | Prüfer |
|---|---|---|
| DEN HAAG | 15-07-1982 | MALHERBE Y.J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82